# EUROPEAN PATENT APPLICATION

(11) **EP 3 251 747 A1**
(43) Date of publication of application: **06.12.2017**
(21) Application number: 16172337.4
(22) Date of filing: 31.05.2016
(51) Int. Cl.: B01J 37/08, B01J 23/75, B01J 35/00, B01J 35/10, B01J 37/14, B01J 37/18, B01J 37/30, B01J 37/02, B01J 23/40, B01J 23/74, B01J 33/00, C07C 29/14, B01J 20/20, C07D 307/20, C07D 307/44, C08G 14/06, C01B 32/318

(54) **PROCESS FOR PREPARING MESOPOROUS CARBON LOADED WITH CATALYTICALLY ACTIVE METAL AND/OR METAL OXIDE NANOPARTICLES FOR THE SELECTIVE TRANSFER HYDROGENATION OF ALPHA-BETA-UNSATURATED ALDEHYDES TO UNSATURATED ALCOHOLS**

(71) Applicant: Studiengesellschaft Kohle mbH, 45470 Mülheim (DE)
(72) Inventor: SCHUETH, Ferdi, 45470 Mülheim an der Ruhr (DE); WANG, Guang-Hui, 45470 Muelheim an der Ruhr (DE)

(57) **Abstract**

The present invention refers to a process for preparing mesoporous carbon loaded with a catalytically active metal and/or metal oxide nanoparticles, the so-obtained mesoporous carbon and the use thereof as a catalyst in a selective transfer hydrogenation process of α,β-unsaturated aldehydes to unsaturated alcohols.

The preparation process containing at least 3 steps: 1) preparing a polymer gel by reacting an aldehyde or an aldehyde forming compound with a hydroxycarboxylic acid in the presence of an aliphatic polyamine and a block copolymer surfactant under hydrothermal conditions. 2) loading this polymer gel with metal ions by ion-exchange. 3) pyrolysis and carbonisation of the loaded polymer gel at elevated temperatures of 400 °C to 1000 °C.

## Description

The present invention refers to a process for preparing mesoporous carbon (MC) loaded with catalytically active metal and/or metal oxide nanoparticles, the so-obtained MC loaded with metal oxide nanoparticles and the use thereof as a catalyst in a transfer hydrogenation process of α,β-unsaturated aldehydes to unsaturated alcohols. Thus, the present invention also refers to a transfer hydrogenation process of α,β-unsaturated aldehydes to unsaturated alcohols catalyzed by metal and/or metal oxide nanoparticles, in particular by Co₃O₄ nanoparticles supported on MC.

The production of high value-added chemicals from biomass is of a major interest to reduce dependence of petroleum-based chemicals. Furan derivatives of furfural (FAL) and 5-hydroxymethylfurfural (HMF), which can be produced from hemicellulose and cellulose respectively, are considered as promising platform molecules to bridge the gap between biomass resources and bio-chemicals since they can be converted into a variety of high value-added chemicals and fuels. Particularly, selective hydrogenation of FAL to furfuryl alcohol (FOL) and HMF to 2,5-bis-(hydroxymethyl)furan (BHMF) has great potential for industrial applications, because FOL and BHMF can be used as precursors in synthesis of polymers, resins and adhesives, and as intermediates in generation of drugs and crown ethers.

However, due to the different functionalities of furan-based α,β-unsaturated aldehydes (e.g., furan ring, C=C and C=O groups), only selective hydrogenation of C=O bond is challenging. Many byproducts were often formed by hydrogenolysis of the -CH=O side chain to -CH₃, or hydrogenation of the furan ring and its opening, leading to low yield of the desired unsaturated alcohols and increasing cost of product purification.

In general, conventional hydrogenation catalysts based on noble or metals (e.g., Pd, Pt, Ru, Rh, Cu, or Ni) show high activity but poor selectivity toward unsaturated alcohols. In order to enhance the selectivity for unsaturated alcohols over such catalysts, additives, stabilizers/ligands, second metal components or functional supports were introduced into the catalytic system or catalysts. In some cases high selectivity toward unsaturated alcohols and high activity were indeed achieved using above methods. However, due to the complexity of reaction mechanism (e.g., competitive/non-competitive, dissociative/non-dissociative adsorption, side reaction), selectivities and activities in these cases can be affected by a series of factors, including the structure/component of catalysts (e.g., particle size, shape, molar ratio of different components) and the reaction conditions (e.g., temperature, pressure and solvents). In other words, dramatic decrease of selectivity and/or activity often occurs because it is very difficult to control these factors precisely, especially in large scale applications.

Therefore, it is necessary to develop a simple method for scale-up of catalyst synthesis; the catalysts must be highly active and selective towards unsaturated alcohols in a simple catalytic system, which is also controllable and scalable. Thus, the design of suitable catalyst and/or catalytic system that facilitate selective hydrogenation of C=O bond in the presence of other functionalities is highly desirable.

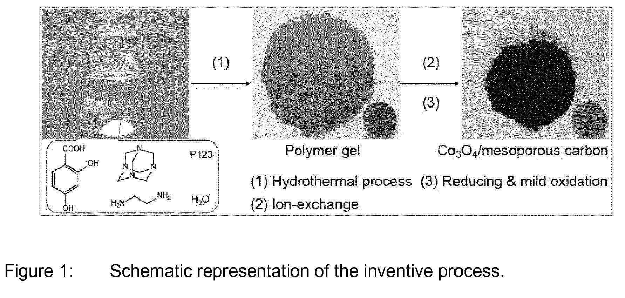

The inventors of the present invention have developed a process in which a mesostructured polymer gel is firstly synthesized using 2,4-dihydroxybenzoic acid (DA) and hexamethylenetetramine (HMT) as polymer precursors and a triblock copolymer such as P123 as surfactant without the addition of a fatty acid salt such as sodium oleate under hydrothermal process conditions (such as at 130 °C for 4 h), and secondly, introducing the Co²⁺ ions homogenously into the mesostructured polymer gel framework in the ion-exchanging step, and thirdly, treating the so-obtained polymer gel loaded with Co²⁺ ions at an elevated temperature for reduction (500 °C, 10% H₂ in argon) first, and then mild oxidation (room temperature, 1% O₂ in argon) so that Co₃O₄ nanoparticles supported on MC (Co₃O₄/MC) are formed after (Figure 1). Said synthetic processes are easy to scale up.

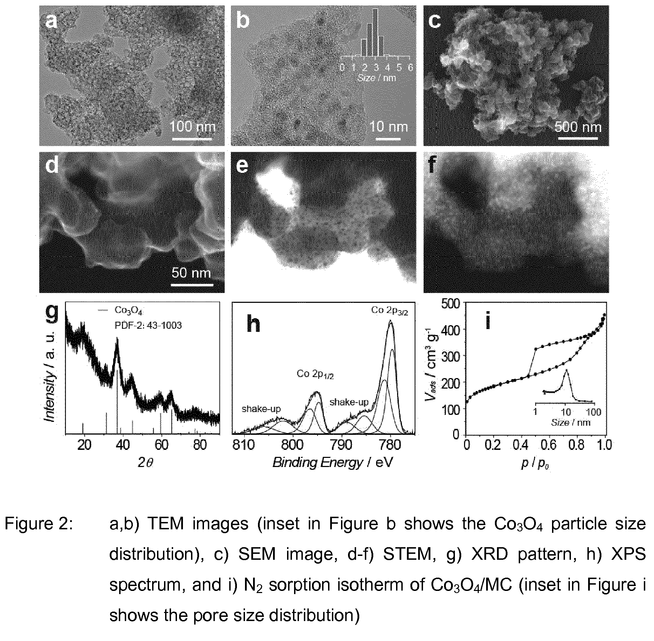

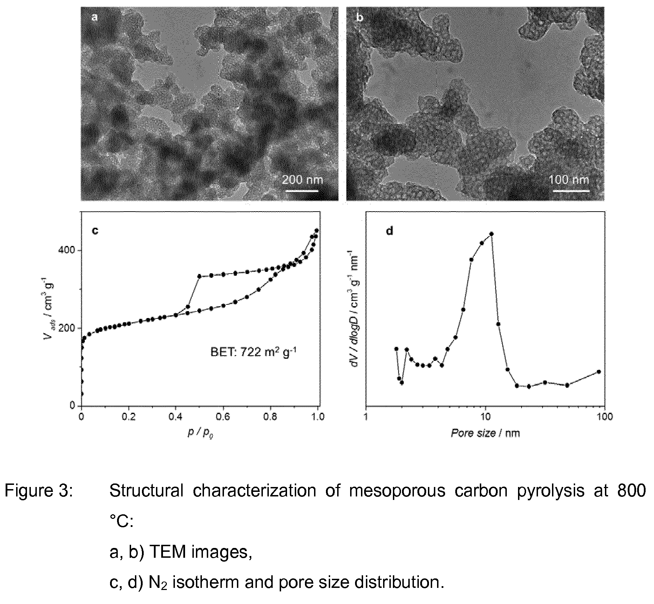

The inventors found out that, in case of Co₃O₄/MC, the MC support is highly mesoporous and the Co₃O₄ nanoparticles with a diameter of ∼3 nm are finely dispersed in the mesoporous framework of MC (Figure 2a,b). Many macropores are also observed in SEM images (Figure 2c), which may be generated from the polymer gel structure. STEM images further confirm that the Co₃O₄ nanoparticles are dispersed very well in the framework of MC with narrow particle size distribution (Figure 2d-f). Most importantly, no bigger Co₃O₄ particles are formed using the present synthesis method. XRD pattern shows the typical reflections corresponding to the Co₃O₄ crystals (PDF-2 entry 43-1003), indicating the formation of Co₃O₄ nanoparticles after the reduction and mild oxidation processes (Figure 2g). XPS spectrum further confirms the formation of Co₃O₄ nanoparticles (Figure 2h). Based on AAS analysis (Atomic absorption spectrometer, Perkin Elmer AAnalyst 200), the Co fraction in Co₃O₄/MC is 15 wt%, corresponding to a Co₃O₄ fraction of 20 wt%. N₂ sorption isotherm of Co₃O₄/MC shows a type-IV curve that is characteristic of mesoporous structure (Figure 2i). In addition, the sorption increase in the high relative pressure region (p/p₀>0.9) indicates the existence of macropores, which is consistent with the SEM observation. The surface area and pore size distribution of Co₃O₄/MC are 642 m² g⁻¹ and 11 nm, respectively. Without introducing Co species, MC with surface area of 722 m² g⁻¹ and pore size of ∼9.5 nm is generated directly after carbonization at 800 °C in argon (Figure 3). This material is a good candidate as catalyst support or adsorbent.

Using traditional impregnation methods, the metal particle size distribution is always broad, which leads to the inefficient use of the Co species because of the size-dependent activity of Co₃O₄. The present synthesis method is suitable to generate Co₃O₄ nanoparticles with narrow size distribution in the range from 2 to 5 nm, preferably 2 to 4 nm, in particular 2.5 to 3.5 nm (∼3 nm) and disperse them in a mesoporous framework of MC homogeneously, which realizes the highly efficient utilization of Co species.

The invention is therefore directed to a process for preparing a mesoporous carbon structure (MC) loaded with catalytically active metal nanoparticles and/or metal oxide nanoparticles, comprising the following steps:
- reacting an aromatic compound having at least one -COOH group and having at least one hydroxyl group with an aldehyde in presence of an aliphatic amine, said amine having 2 to 12 carbon atoms and at least two amine groups, and a surfactant under hydrothermal conditions whereby a mesostructured polymer gel is obtained,
- treating the obtained mesostructured polymer gel with a solution of a metal salt or with a mixture of different salts in an ion-exchanging step whereby a mesostructured polymer gel loaded with metal ions is obtained,
- treating the obtained polymer gel loaded with metal ions in a protective gas atmosphere at an elevated temperature in the range of 400 °C to 1000 °C whereby a mesoporous carbon structure loaded with catalytically active metal nanoparticles and/or metal oxide nanoparticles is obtained.

In the process, the aromatic compound is linked with the aldehyde in the presence of the amine and a mesostructured polymer gel is obtained. The obtained polymer gel is then loaded with metal ions which may finally be present as metal particles and/or metal oxide particles, depending on the metal. Depending on the reaction conditions, a noble metal will be present as metal particles whereas non-noble metals will be present as a metal which might optionally be further oxidized to a corresponding metal oxide and which may be obtainable by treating the obtained mesoporous carbon structure in a gas atmosphere with an oxygen content under conditions under the MC support is not oxidized, preferably in a temperature range from 20 °C to 200 °C, more preferred at a temperature between 20 °C and 100 °C whereby a mesoporous carbon network structure loaded with catalytically active metal and/or metal oxide nanoparticles is obtained.

As an alternative process, the mesoporous carbon structure loaded with catalytically active metal and/or metal oxide nanoparticles may be obtained by reacting an aromatic compound having at least one -COOH group and having at least one hydroxyl group with an aldehyde in presence of an aliphatic amine, said amine having 2 to 12 carbon atoms and at least two amine groups, and a surfactant under hydrothermal conditions whereby a mesostructured polymer gel is obtained,
treating the obtained polymer gel in a protective gas atmosphere at an elevated temperature in the range of 400 °C to 1000 °C, whereby a mesoporous carbon structure is obtained,
impregnating the obtained mesoporous carbon structure with a solution of a metal salt or with a mixture of different salts whereby a mesoporous carbon structure loaded with metal ions is obtained;
treating the obtained mesoporous carbon structure loaded with metal ions in a protective gas atmosphere at an elevated temperature in the range of 200 °C to 1000 °C, optionally in the presence of hydrogen whereby a mesoporous carbon structure loaded with catalytically active metal nanoparticles and/or metal oxide nanoparticles is obtained.

In the latter process, an empty mesoporous structure is prepared first, and in a further step loaded with the metal ions which are then converted to the metal and/or metal oxide particles.

In the inventive process, the aromatic compound having at least one -COOH group and having at least one hydroxyl group may be selected from aromatic hydrocarbons such as phenyl, naphthyl, or anthryl, and can be, as example, dihydroxy benzoic acid. The aromatic compound may preferably have up to three -COOH groups and up to three hydroxyl groups whereby an aromatic compound having three or four functional groups, with at least one -COOH group and at least one hydroxyl group and the other(s) being selected from -COOH and hydroxyl, is preferred.

The aldehyde may be selected from an aliphatic C₁ to C₁₂ hydrocarbon aldehyde such as formaldehyde, paraformaldehyde, furfuraldehyde, acetaldehyde, crotonaldehyde, an aromatic aldehyde such as benzaldehyde or substituted derivatives thereof or a compound which can be decomposed into formaldehyde such as hexamethylenetetramine and urea. The expression "aldehyde compound" is intended to indicate one -CHO-unit used for bridging the aromatic compound.

The aliphatic amine serves as a linker between the -COOH group of two aromatic compounds or as base to neutralize the acidity of the aromatic compound with - COOH group and may be any aliphatic hydrocarbon having 2 to 12 carbon atoms and having at least two, three or four amino groups. Examples are ethylene diamine, propylene diamine, hexane diamine, octane triamine, or mixtures of different aliphatic amines. Preferably, the aliphatic amine is soluble in or miscible with water.

As surfactant, an amphiphilic triblock copolymer such as a poly(ethylene oxide)-poly(alkylene oxide)-poly(ethylene oxide) polymer is preferably used in the inventive process. The central alkylene oxide moiety has at least three carbon atoms. Thus, amphiphilic triblock copolymer of poly(ethylene oxide)-poly(propylene oxide)-poly(ethylene oxide) are most preferred. Preferably, said amphiphilic triblock copolymers are used as the only surfactant and no further surface-active materials are added in the hydrothermal treatment.

The hydrothermal treatment is generally carried out in an aqueous solution at a temperature in the range of 60 °C or more up to 200 °C, preferably under autogenous pressure in an autoclave.

During the hydrothermal treatment, the aromatic compound having at least one -COOH group and having at least one hydroxyl group is reacted with the aldehyde compound in a kind of phenol-aldehyde resin formation process in the presence of the amphiphilic triblock copolymer as surfactant, thus leading to the mesostructured polymer gel which is then optionally comminuted or grinded and optionally washed, preferably with water, and then treated with the metal salt. In the next process step, the mesostructured polymer gel is converted, in a protective gas atmosphere at an elevated temperature, to the MC loaded with metal and/or metal oxide particles.

In the hydrothermal treatment step, the aromatic compound having at least one - COOH group and having at least one hydroxyl group is preferably polymerized with the compound producing the aldehyde group in molar ratios of 1 to 3 (aromatic compound) to 1 to 5 (of reacting aldehyde group) in the presence of the aliphatic amine (0.5 to 1.5) and surfactant (0.03 to 0.09). In a very simple ratio, the compounds as indicated before are used in a ratio of 2 : 3: 1 : 0.06. In a particular embodiment, the compound producing the -CHO group can be preferably used in over-stoichiometric amounts in order to ensure complete crosslinking of the aromatic molecules.

If needed, the pH of the reaction mixture in the hydrothermal treatment is adjusted to a weakly basic range from 7 to 12.

In the next step, the obtained mesostructured polymer gel is treated with a solution of a metal salt in an ion-exchanging step whereby a mesostructured polymer gel loaded with metal ions is obtained.

As a catalytically active metal, any metal is suitable and is selected from Ti, V, Cr, Mn, Fe, Co, Ni, Cu, Zn, Al, Mo, Se, Sn, Pt, Ru, Rh, Zr, Hf, Re, Pd, W, Ir, Os, Rh, Nb, Ta, Pb, Bi, Au, Ag, Sc, Y and preferably from Fe, Co, Ni, Pt, Ru, Rh and Pd. Co is most preferred.

The metal may be applied in the form of a salt and mixtures thereof, preferably in the form of a solution of a metal salt or a mixture thereof, which are later in the process converted into the metal, metal alloy or metal oxides, depending on the used metal.

In the next step, the obtained polymer gel loaded with metal ions is treated in a protective gas atmosphere, preferably at an elevated temperature in the range of 400 °C to 1000 °C whereby a MC loaded with metal and/or metal oxide particle is obtained. As a protective gas atmosphere, argon, nitrogen or mixtures thereof, optionally in admixture with hydrogen, is preferably used, preferably in a volume ratio of 1 to 10 % hydrogen.

In the additional step, the obtained mesoporous carbon structure is treated in a gas atmosphere with an oxygen content under conditions which are not oxidizing the MC support, preferably in a temperature range from 20 °C to 200 °C, more preferred at a temperature between 20 °C and 100 °C whereby a mesoporous carbon network structure loaded with catalytically active metal and/or metal oxide nanoparticles is obtained.

The present invention also refers to the mesoporous carbon loaded with metal oxide nanoparticles, obtainable according to the process of the present invention, in particular to the mesoporous carbon loaded with metal oxide nanoparticles, wherein the metal oxide nanoparticles have a particle size in the range of 1 to 10 nm, preferably 2 to 6 nm, in particular 2.5 to 4.5 nm, for Co₃O₄ in particular from 2 to 5 nm, preferred 2 to 4 nm and most preferred from 2.5 to 3.5 nm.

Said mesoporous carbon supported Co₃O₄ nanoparticles can be preferably used as a catalyst, in particular as a catalytically active material in a process for transfer hydrogenation of α,β-unsaturated aldehydes in the presence of the inventive catalyst and a H-donor, for example a secondary alcohol such as iso-propanol, to unsaturated alcohols as represented in the following scheme:

R¹-CR²=CR³-CH=O → R¹-CR²=CR³-CH₂-OH,

wherein R¹ to R³ may be the same or different and may be selected each from C₁ to C₂₀ straight chain, branched chain or cyclic aliphatic hydrocarbons, optionally having one or more heteroatoms such as O, N, or S, in the chain or ring or unsaturated bonds such as C₁-C₂₀-alkyl, C₂-C₂₀-alkenyl or C₂-C₂₀-alkinyl, C₃-C₈-heterocycloalkyl or C₆ to C₂₀ aromatic hydrocarbon and partially arene-hydrogenated forms such as aryl, aryl-(C₁-C₆)-alkyl, heteroaryl-(C₁-C₆)-alkyl, each hydrocarbon optionally being substituted by one or more groups selected from C₁ to C₂₀ straight chain, branched chain or cyclic aliphatic hydrocarbons, optionally having one or more unsaturated bonds such as C₁-C₂₀-alkyl, C₂-C₂₀-alkenyl or C₂-C₂₀-alkinyl, or C₆ to C₂₀ aromatic hydrocarbon and partially arene-hydrogenated forms such as aryl, aryl-(C₁-C₆)-alkyl, heteroaryl-(C₁-C₆)-alkyl or heterosubstituents, or wherein one of R¹ or R² may form a ring with R³, optionally having one or more heteroatoms in the ring, and the other of R¹ or R² is as defined before.

The invention is further illustrated by the attached drawings. In said drawings,
Figure 1 shows a schematic representation of the inventive process;
Figure 2 shows a,b) TEM images (inset in Figure b shows the Co₃O₄ particle size distribution), c) SEM image, d-f) STEM, g) XRD pattern, h) XPS spectrum, and i) N₂ sorption isotherm of Co₃O₄/MC (inset in Figure i shows the pore size distribution);
Figure 3 shows the structural characterization of mesoporous carbon pyrolysis at 800 °C: a, b) TEM images, and c, d) N₂ isotherm and pore size distribution;
Figure 4 shows TEM images and XRD patterns of Co₃O₄ materials: a, b) Co₃O₄-nanocasting, c, d) Co₃O₄-6 nm, and e, f) Co₃O₄-17 nm;
Figure 5 shows a) catalytic performances for the transfer hydrogenation of cinnamaldehyde and citral over Co₃O₄/MC, b) the recycling results for transfer hydrogenation of FAL over Co₃O₄/MC.
Figure 6 shows a,b) TEM images of Co₃O₄/MC after 6 runs, c) XRD patterns and d) N₂ isotherms of Co₃O₄/MC before and after 6 runs.

### Experimental Section:

### Synthesis of Co₃O₄ supported on mesoporous carbon (Co₃O₄/MC):

In a typical synthesis, 3.08 g of 2,4-dihydroxybenzoic acid, 0.6 g of ethylenediamine, 0.934 g of hexamethylentetramine (HMT) and 3.5 g of Pluronic P123 were dissolved in 80 mL of H₂O. The solution was transferred into a teflon-lined stainless steel autoclave of 120 mL capacity, sealed and heated up to 130 °C and kept at that temperature for 4 h. Afterwards, the autoclave was allowed to cool down to room temperature. The polymer gel product was mashed and washed three times with deionized water. After dring at 50 °C over night, ∼5.77 g of polymer product was obtained. The polymer product was re-dispersed in 120 mL of solution (96 mL of H₂O and 24 mL of ammonium hydroxide solution (28.0-30.0%)) containing 4.62 mmol (1.345 g) of Co(NO₃)₂·6H₂O, stirred at 50 °C for 6 h. Then, the product was collected by filtration, washed three times with deionized water and dried at 50 °C under vacuum for 8 h. Finally, ∼1.64 g of Co₃O₄/MC was obtained by pyrolysis under H₂/Ar (5%/95%) atmosphere. The pyrolysis procedure used here was as follows: the sample was heated to 400 °C with a rate of 2 °C min⁻¹ and kept at that temperature for 3 h, then heated to 500 °C with a rate of 1 °C min⁻¹ and kept at that temperature for 2 h. Afterwards, the sample was allowed to cool to room temperature and passivate in a flow of 1 % oxygen in argon for 2 h.

For synthesis of MC without the metal load, the polymer gel product was directly carbonized by heating up to 800 °C with a heating rate of 2 °C min⁻¹ and holding at that temperature for 3 h under argon atmosphere.

### Synthesis of Co₃O₄-nanocasting, Co₃O₄-6 nm and Co₃O₄-17 nm:

The Co₃O₄-nanocasting was prepared completely according to the method reported in literature. Co₃O₄-6 nm and Co₃O₄-17 nm were synthesized via a hydrothermal approach according to literature.^{[2]} For the synthesis of 6 nm Co₃O₄ nanoparticles, 0.5 g of Co(CH₃COO)₂·4H₂O was first dissolved in 25 mL of ethanol. Then 2.5 mL of 25% ammonia was added under vigorous stirring. After 10 min the obtained slurry was transferred to a teflon-lined stainless steel autoclave of 45 mL capacity, sealed and maintained at 150 °C for 3 h. Afterwards the resulting black solid was collected by centrifuge, washed intensively with ethanol and water, and finally dried under vacuum at 50 °C for 8 h. For the synthesis of 17 nm Co₃O₄ nanoparticles, the solvent was changed to a mixture of water (10 mL) and ethanol (15 mL). The rest of experimental conditions remained the same.

### Transfer hydrogenation of substrates

For a typical run, 1 mmol of substrates, 10 mL of 2-propanol, 50 mg of Co₃O₄/MC and a magnet bar were placed in a glass vial (20 mL). The vial was flushed with argon and then tightly closed. The experiment was performed at 120 °C under magnetic stirring of 800 rpm in a stainless steel heating block. A small volume of sample (∼0.1 mL) was periodically withdrawn and analyzed by GC-MS. 1,6-hexandiol was chosen as internal standard for FAL and HMF system, while n-decane was used as internal standard for cinnamaldehyde and citral. When increasing reaction temperature to 140 °C and 160 °C, the experiments were carried out in a stainless steel autoclave reactor with volume of 20 mL. The rest of experimental conditions remained the same.

### Characterization:

Transmission electron microscopy (TEM), scanning transmission electron microscopy (STEM) and scanning electron microscopy (SEM) analyses were carried out with Hitachi HF-2000 and Hitachi S-5500 microscopes, respectively. All samples were prepared by dipping carbon-coated copper grids into the ethanol solutions with the solid products and drying them at room temperature. Powder X-ray diffraction (XRD) was performed on a Stoe STADI P diffractometer operating in reflection mode with Cu Kα radiation using a secondary graphite monochromator.
Nitrogen sorption isotherms were measured with a Micromeritics ASAP 2010 adsorption analyzer at 77 K. Prior to the measurements, the sample was degassed at a temperature of 250 °C for 6 h.
The specific surface areas were calculated from the adsorption data in the relative pressure range of 0.05 to 0.3 using the Brunauer-Emmett-Teller (BET) method. Pore size distributions were determined with the Barrett-Joyner-Halenda (BJH) method from the adsorption branch (desorption data which are normally recommended, can be influenced by network percolation or cavitation effects). The total pore volume was estimated from the amount adsorbed at a relative pressure of 0.97.
X-ray photoelectron spectroscopy (XPS) measurements were carried out with a Kratos HSi spectrometer with a hemispherical analyzer. The monochromatized Al Kα X-ray source (E=1486.6 eV) was operated at 15 kV and 15 mA. An analyzer pass energy of 40 eV was applied for the narrow scans. Hybrid mode was used as lens mode. The base pressure during the experiment in the analysis chamber was 4×10⁻⁷ Pa. To account for charging effects of carbonized samples, the binding energy values were referred to C 1 s at 284.5 eV.
Elemental analysis was carried out at Mikrolab Kolbe (Höhenweg 17, D-45470, Mülheim an der Ruhr) by AAnalyst 200 Atomic Absorption Spectrometer (AAS).

**Table S1. Selective hydrogenation of α,β-unsaturated aldehydes^{[a].}**

| Entry | Substrate | Catalyst | Temp. (°C) | Tim. (h) | Product | Conv. (%) | Sel. (%) | Rate (mmol g_{Co3O4}⁻¹ h⁻¹)^{[b]} |
|---|---|---|---|---|---|---|---|---|
| 1 | 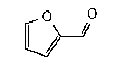 | Co₃O₄/MC | 120 | 8 | 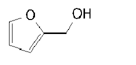 | 100 | 97 | 37.5 (64%, 2h) |
| 2 | | | 140 | 3 | | 100 | 97 | 148.2 (63%, 0.5h) |
| 3 | | | 160 | 1 | | 100 | 98 | 401.4 (57%, 0.167h) |
| 4 | | Co₃O₄-nanocasting | 120 | 24 | | 17 | 99 | 0.32 (17%, 24h) |
| 5 | | Co₃O₄-6nm | 120 | 24 | | 46 | 97 | 2.29 (22%, 4h) |
| 6 | | Co₃O₄-17nm | 120 | 24 | | 15 | 99 | 0.26 (15%, 24h) |
| 7 | 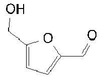 | Co₃O₄/MC | 120 | 48 | 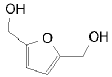 | 100 | 97 | 11.1 (64%, 6h) |
| 8 | | | 140 | 12 | | 100 | 98 | 57.5 (63%, 1h) |
| 9 | | | 160 | 6 | | 100 | 99 | 133.3 (73%, 0.5h) |
| 10 | | Co₃O₄-nanocasting | 120 | 48 | | 19 | 99 | 0.16 (19%, 48h) |
| 11 | | Co₃O₄-6nm | 120 | 48 | | 37 | 99 | 0.65 (19%, 12h) |
| 12 | | Co₃O₄-17nm | 120 | 48 | | 11 | 99 | 0.10 (11%, 48h) |
| 13^{[c]} | 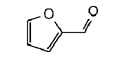 | Co₃O₄/MC | 160 | 4 | 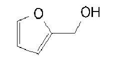 | 100 | 97 | - |
| 14 | 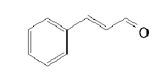 | Co₃O₄/MC | 120 | 24 | 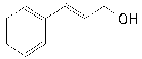 | 99 | 98 | - |
| 15 | 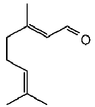 | Co₃O₄/MC | 120 | 48 | 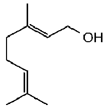 | 99 | 95 | - |

| | | | | | | | | |
|---|---|---|---|---|---|---|---|---|
| [a] Reaction conditions: 1 mmol substrate, 10 mL 2-propanol, 50 mg Co₃O₄/MC (25 mg for Co₃O₄-nanocasting, Co₃O₄-6 nm and Co₃O₄-17 nm). All results were obtained from GC testing. [b] The numbers in parentheses are the conversion and the reaction time for reaction rate estimation. [c] Scale-up reaction: 1.16 g furfural, 100 mL 2-propanol, 500 mg Co₃O₄/MC. | | | | | | | | |

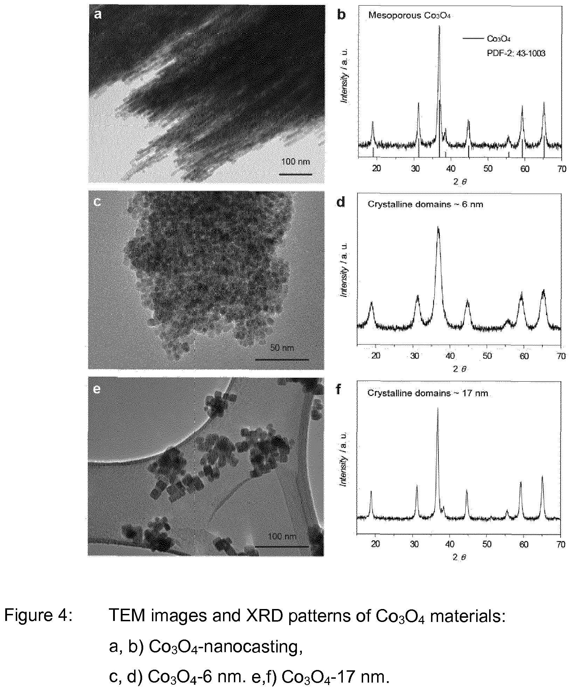

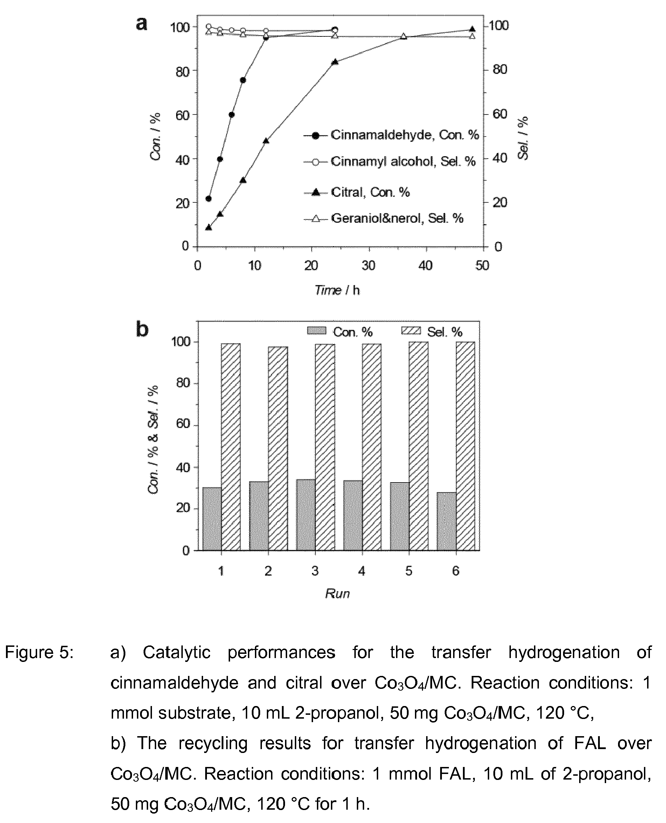

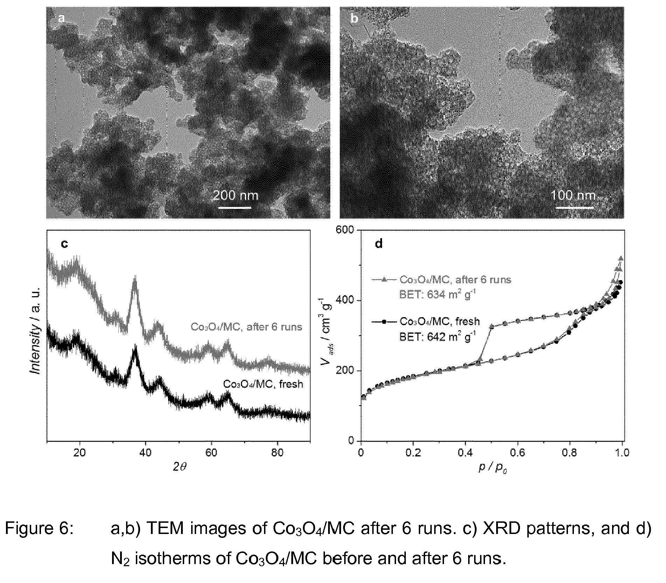

As shown above, the inventors have developed a simple and scalable method, including steps of hydrothermal process, ion-exchanging and reducing/mild oxidizing, to synthesize metal oxide nanoparticles such as Co₃O₄ nanoparticles supported in the framework of the mesoporous carbon network. Benefiting from the ion-exchange process, where Co²⁺ ions can be introduced into the polymer framework homogenously, the Co₃O₄ nanoparticles with a diameter of ∼3 nm were finely dispersed in the framework of MC after reduction and mild oxidation processes. The as-obtained Co₃O₄/MC is more efficient than Co₃O₄-nanocasting, Co₃O₄-6 nm and Co₃O₄-17 nm (Figure 4) as catalyst for the transfer hydrogenation of α,β-unsaturated aldehydes (Table 1, Figure 5a). The selectivities towards unsaturated alcohols over Co₃O₄/MC are always higher than 97% at full conversion. Furthermore, the catalyst after reaction was filtrated and washed with 2-propanol, followed by drying and treating under H₂/Ar at 300 °C for 2 h to remove residues from the surface of the used Co₃O₄/MC. In this way, the Co₃O₄/MC catalyst was recycled at least six times without loss of activity, indicating a high stability of Co₃O₄/MC under the reaction conditions (Figure 5b, 6). The gram-scale preparation of furfural alcohol over Co₃O₄/MC indicates that such catalytic system is scalable without pressure issue (Table 1, entry 13). The as-obtained furfuryl alcohol can be used as polymer precursor directly without further purification to synthesize ordered mesoporous carbon (CMK-5).

Therefore, the present catalytic system for transfer hydrogenation of α,β-unsaturated aldehydes over Co₃O₄/MC has the potential to be utilized in industry. In addition, the synthesis methodology of Co₃O₄/MC, which is also easy to scale up, may be further extended to design other metal or metal oxide catalysts supported on MC.

## Claims

1. Process for preparing a mesoporous carbon structure loaded with catalytically active metal nanoparticles and/or metal oxide nanoparticles, comprising the following steps:
- reacting an aromatic compound having at least one -COOH group and having at least one hydroxyl group with an aldehyde in presence of an aliphatic amine, said amine having 2 to 12 carbon atoms and at least two amine groups, and a surfactant under hydrothermal conditions whereby a mesostructured polymer gel is obtained,
- treating the obtained mesostructured polymer gel with a solution of a metal salt or with a mixture of different salts in an ion-exchanging step whereby a mesostructured polymer gel loaded with metal ions is obtained,
- treating the obtained polymer gel loaded with metal ions in a protective gas atmosphere at an elevated temperature in the range of 400 °C to 1000 °C whereby a mesoporous carbon structure loaded with catalytically active metal nanoparticles and/or metal oxide nanoparticles is obtained.

2. Process for preparing a mesoporous carbon structure loaded with catalytically active metal nanoparticles and/or metal oxide nanoparticles according to claim 1, comprising the additional step of:
- treating the obtained mesoporous carbon structure in a gas atmosphere with an oxygen content under conditions which are not oxidizing the MC support, preferably in a temperature range from 20 °C to 200 °C, more preferred at a temperature between 20 °C and 100 °C whereby a mesoporous carbon network structure loaded with catalytically active metal and/or metal oxide nanoparticles is obtained.

3. Process for preparing a mesoporous carbon structure loaded with catalytically active metal and/or metal oxide nanoparticles, comprising the following steps:
- reacting an aromatic compound having at least one -COOH group and having at least one hydroxyl group with an aldehyde in presence of an aliphatic amine, said amine having 2 to 12 carbon atoms and at least two amine groups, and a surfactant under hydrothermal conditions whereby a mesostructured polymer gel is obtained,
- treating the obtained polymer gel in a protective gas atmosphere at an elevated temperature in the range of 400 °C to 1000 °C, whereby a mesoporous carbon structure is obtained,
- impregnating the obtained mesoporous carbon structure with a solution of a metal salt or with a mixture of different salts whereby a mesoporous carbon structure loaded with catalytically active metal and/or metal oxide nanoparticles is obtained;
- treating the obtained mesoporous carbon structure loaded with metal ions in a protective gas atmosphere at an elevated temperature in the range of 200 °C to 1000 °C, optionally in the presence of hydrogen whereby a mesoporous carbon structure loaded with catalytically active metal nanoparticles and/or metal oxide nanoparticles is obtained.

4. Process for preparing a mesoporous carbon structure according to any of claims 1 to 3 wherein the surfactant is an amphiphilic block copolymer.

5. Process for preparing a mesoporous carbon structure according to any of claims 1 to 3 wherein the surfactant is a poly(ethylene oxide)-poly(alkylene oxide)-poly (ethylene oxide) polymer wherein the alkylene oxide has at least three carbon atoms.

6. Process for preparing a mesoporous carbon structure according to any of claims 1 to 3 wherein the surfactant is a poly(ethylene oxide)-poly(propylene oxide)-poly (ethylene oxide) polymer.

7. Process for preparing a mesoporous carbon structure according to any of claims 1 to 6 wherein the metal is selected from Ti, V, Cr, Mn, Fe, Co, Ni, Cu, Zn, Al, Mo, Se, Sn, Pt, Ru, Rh, Pd, W, Ir, Os, Rh, Zr, Hf, Re, Nb, Ta, Pb, Bi, Au, Ag, Sc, Y, preferably from Fe, Co, Ni, Pt, Ru, Rh and Pd, more preferred from Fe, Co, Ni, and most preferred Co.

8. Mesoporous carbon structure loaded with metal nanoparticles and/or metal oxide nanoparticles, obtainable according to the process of any of claims 1 to 7.

9. Mesoporous carbon structure loaded with metal nanoparticles and/or metal oxide nanoparticles, obtainable according to the process of any of claims 1 to 7 wherein the metal nanoparticles and/or metal oxide nanoparticles have a particle size in the range of 1 to 10 nm, preferably 2 to 6 nm, in particular 2.5 to 4.5 nm.

10. Use of the mesoporous carbon structure of claim 8 or 9 as a catalyst.

11. Use of the mesoporous carbon structure of claim 8 or 9 as a catalytically active material in a process for transfer hydrogenation of α,β-unsaturated aldehydes to unsaturated alcohols in the presence of a H-donor as represented in the following scheme:
R¹-CR²=CR³-CH=O → R¹-CR²=CR³-CH₂-OH,
wherein R¹ to R³ may be the same or different and may be selected each from C₁ to C₂₀ straight chain, branched chain or cyclic aliphatic hydrocarbons, optionally having one or more heteroatoms such as O, N, or S, in the chain or ring or unsaturated bonds such as C₁-C₂₀-alkyl, C₂-C₂₀-alkenyl or C₂-C₂₀-alkinyl, C₃-C₈-heterocycloalkyl or C₆ to C₂₀ aromatic hydrocarbon and partially arene-hydrogenated forms such as aryl, aryl-(C₁-C₆)-alkyl, heteroaryl-(C₁-C₆)-alkyl, each hydrocarbon optionally being substituted by one or more groups selected from C₁ to C₂₀ straight chain, branched chain or cyclic aliphatic hydrocarbons, optionally having one or more unsaturated bonds such as C₁-C₂₀-alkyl, C₂-C₂₀-alkenyl or C₂-C₂₀-alkinyl, or C₆ to C₂₀ aromatic hydrocarbon and partially arene-hydrogenated forms such as aryl, aryl-(C₁-C₆)-alkyl, heteroaryl-(C₁-C₆)-alkyl or heterosubstituents, or wherein one of R¹ or R² may form a ring with R³, optionally having one or more heteroatoms in the ring, and the other of R¹ or R² is as defined before.
